(19) Europäisches Patentamt
European Patent Office
Office européen des brevets

(11) Veröffentlichungsnummer: **0 415 250 A1**

(12) # EUROPÄISCHE PATENTANMELDUNG

(21) Anmeldenummer: **90116038.2**

(22) Anmeldetag: **22.08.90**

(51) Int. Cl.5: **C07C 17/42**, B41M 5/165

(30) Priorität: **26.08.89 DE 3928319**

(43) Veröffentlichungstag der Anmeldung: **06.03.91 Patentblatt 91/10**

(84) Benannte Vertragsstaaten: **BE DE DK ES FR GB GR IT LU NL**

(71) Anmelder: **HOECHST AKTIENGESELLSCHAFT**
**Postfach 80 03 20**
**W-6230 Frankfurt am Main 80(DE)**

(72) Erfinder: **Landau, Helmut**
**Altvaterstrasse 4**
**W-8906 Gersthofen(DE)**

(54) **Verfahren zur Stabilisierung von chlorierten Paraffinen.**

(57) Chlorierte Paraffine, welche Fettaminoxethylate als Stabilisatoren enthalten, eignen sich hervorragend als Lösemittel für die Farbbildner in chemisch reagierenden Durchschreibepapieren.

EP 0 415 250 A1

# VERFAHREN ZUR STABILISIERUNG VON CHLORIERTEN PARAFFINEN

Die Erfindung bezieht sich auf die Stabilisierung von Chlorparaffinen, die als Lösemittel für die Farbbildner von chemisch reagierenden Durchschreibepapieren verwendet werden.

Die heute meist verwendeten kohlefreien Durchschreibepapiere arbeiten nach dem Prinzip der Farbbildung durch Reaktion einer elektronenabgebenden organischen Verbindung, dem sogenannten Farbbildner, welcher keine oder nur eine äußerst geringe Eigenfarbe besitzt, mit einer elektronenaufnehmenden Substanz, dem sogenannten Farbentwickler.

Die üblichen drucksensitiven Kopierpapiere bestehen aus einer Kombination eines Papieroberbogens, auf dessen Rückseite die mikroverkapselte Emulsion des Farbbildners aufgetragen ist (Geberschicht = CB-Schicht = Coated Back). Auf dem Papierunterbogen ist die elektronenaufnehmende Substanz für die Farbentwicklung aufgetragen (Nehmerschicht = CF-Schicht = Coated Front). Bei einem Durchschreibesatz mit 3 oder mehr Blättern wird mit Zwischenblättern gearbeitet, die auf der Oberseite die CF-Schicht und auf der Unterseite die CB-Schicht tragen (CFB-Zwischenblätter).

Durch Druck oder Schlag werden die Mikrokapseln, in welchen der gelöste Farbbildner eingeschlossen ist, zerstört und die Farbbildnerlösung sofort von der saugfähigen Nehmerschicht absorbiert und fixiert.

Als elektronenaufnehmende Substanzen oder Farbentwickler für die CF-Schicht sind bekannt:
- sauer aktivierte Aluminium-Schichtsilikate auf
Montmorillonit-Basis, aktivierte Clay-Qualitäten, wie beispielsweise Silton, Bentonite, Attapulgite oder synthetische Produkte, wie beispielsweise behandelte Anhydrite,Aluminiumsilikate, Magnesiumsilikate
- spezifische Zinksalicylat-Verbindungen
- phenolische Harze, wie z.B. Kondensationsprodukte von Paraphenylphenol, Para-Alkylphenolen mit Formaldehyd.

Als Farbbildner für die CB-Schicht werden beispielsweise Kristallviolettlacton, Malachitgrünlacton, Benzoylleucomethylenblau, Rhodamin-B-lacton u.ä. Substanzen verwendet. In den meisten Fällen wird ein Gemisch verschiedener Farbbildner eingesetzt.

Als Lösemittel für die Farbbildner können viele organische Flüssigkeiten verwendet werden, wobei die üblichen Substanzen modifizierte Terphenyle oder alkylierte Naphtaline oder Diphenyle in Kombination mit verschiedenen Kerosintypen sind.

Die Verwendung von geradkettigen chlorierten Paraffinkohlenwasserstoffen mit 6 bis 18 Kohlenstoffatomen und Chlorgehalten von 20 bis 60 Gew.-% als Lösemittel für die Farbbildner ist bekannt (vgl. GB 1 296 477). Chlorparaffine haben die gewünschten Eigenschaften, die an das Lösemittel gestellt werden. Sie sind gute Lösemittel für die Farbbildner, sie sind verfügbar in allen Viskositätsklassen durch entsprechende Variation der C-Kettenlängen der Paraffine und des Chlorierungsgrades. Sie besitzen keinen störenden Geruch. Chlorierte Paraffine verringern die Farbbildungsempfindlichkeit der CF-Schicht nicht.

Es hat sich jedoch gezeigt, daß eine Stabilisierung mit den üblicherweise in chlorierten Paraffinen eingesetzten epoxidierten Ölen z.B. epoxidiertem Sojabohnenöl oder epoxidgruppenhaltigen Harzen allein nicht ausreicht, eine Farbbildung der Farbbildner-Lösung selbst bei Raumtemperatur zu verhindern. Dieser Effekt macht sich durch eine Schleierbildung auf den Durchschlagpapieren bemerkbar, die durch erhöhte Temperatur oder bei Sonneneinstrahlung noch verstärkt wird. Auch die Intensität des Schriftbildes leidet unter dieser unerwünschten Farbbildung.

Die Aufgabe bestand daher darin, eine Stabilisierung zu finden, welche die vorstehend genannten Nachteile nicht aufweist.

Es wurde gefunden, daß durch Zusatz bestimmter Fettaminethoxylate zu dem chlorierten Paraffin die vorzeitige Verfärbung der Farbbildnerlösung verhindert werden kann.

Die Erfindung betrifft somit ein Verfahren zum Stabilisieren eines chlorierten, im wesentlichen geradkettigen Paraffins mit 7 bis 35 Kohlenstoffatomen und einem Chlorgehalt von 10 bis 72 Gew.-%, dadurch gekennzeichnet, daß man dem chlorierten Paraffin 0,1 bis 3 Gew.-%, bezogen auf das chlorierte Paraffin, mindestens eines Fettaminoxethylats zusetzt.

Die Erfindung betrifft weiterhin ein chloriertes, im wesentlichen geradkettiges Paraffin mit 7 bis 35 Kohlenstoffatomen und einem Chlorgehalt von 10 bis 72 Gew.-%, enthaltend 0,1 bis 3 Gew.-%, bezogen auf das chlorierte Paraffin, mindestens eines Fettaminoxethylats und seine Verwendung.

Die erfindungsgemäß zu stabilisierenden chlorierten Paraffine sind Chlorierungsprodukte von im wesentlichen geradkettigen Paraffinen mit 7 bis 35 Kohlenstoffatomen im Molekül, bevorzugt von technischen Paraffinschnitten mit beispielsweise 10 bis 13, 14 bis 17, 18 bis 24 und so weiter Kohlenstoffatomen. Der Chlorgehalt dieser Chlorierungsprodukte beträgt 10 bis ca. 72 Gew.-%.

Die erfindungsgemäß zu verwendenden Fettaminoxethylate werden erhalten durch Einwirkung von

Ethylenoxid auf Fettamine. Die Umsetzung kann summarisch vereinfacht durch folgendes Schema dargestellt werden:

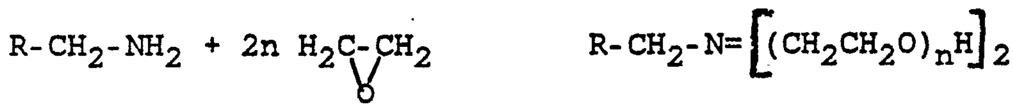

**Fettamin Ethylenoxid Fettaminoxethylat**

2n = Zahl der angelagerten Moleküle Ethylenoxid.

Durch die Anlagerung von Ethylenoxid entstehen aus den Fettaminen konstitutionell zweiwertige Alkohole mit endständigen Hydroxylgruppen. Fettaminethoxylate sind basischer Natur und reagieren in wäßriger Lösung alkalisch. Ihre physikalisch-chemischen Eigenschaften, insbesondere die grenzflächenaktiven Eigenschaften, werden maßgeblich bestimmt durch das Verhältnis des hydrophoben Fettaminrestes zur hydrophilen Polyglykolkette im Gesamtmolekül. Die Länge der Polyglykolkette ist gegeben durch die Anzahl der je Molekül Fettamin angelagerten Moleküle Ethylenoxid. Da sowohl die Art des Fettamins als auch die Menge Ethylenoxid beliebig gewählt werden kann, gibt es viele Möglichkeiten zur Abwandlung des hydrophil/lipophilen Gleichgewichts.

Bevorzugte Fettamine sind:
Cocosfettamin - gesättigte $C_8$-$C_{18}$-Fettamine, vorwiegend $C_{12}$-$C_{14}$ Stearylamin - ungesättigte $C_{16}$-$C_{18}$-Fettamine Oleylamin - überwiegend ungesättigtes $C_{18}$-Fettamin Talgfettamin - überwiegend ungesättigtes $C_{16}$-$C_{20}$-Fettamin

Die Anzahl der angelagerten Moleküle Ethylenoxid beträgt 2 bis 25, vorzugsweise 2 bis 25 Moleküle AeO je Molekülfettamin. Je nach verwendetem Fettamin und Anzahl der Ethylenoxidmoleküle (Oxethylierungsgrad) sind die Fettaminoxethylate mehr wasserlöslich oder mehr löslich in Kohlenwasserstoffen oder chlorierten Paraffinen.

Das Fettaminoxethylat ist im chlorierten Paraffin in einer Menge von 0,1 bis 3, vorzugsweise 0,5 bis 2 Gew.-%, bezogen auf das chlorierte Paraffin, enthalten.

Das verwendete chlorierte Paraffin kann zusätzlich auch die bekannten Stabilisatoren, beispielsweise epoxidierte Öle, wie epoxidiertes Sojabohnenöl, oder epoxidierte Harze, beispielsweise cycloaliphatisches Diepoxid, in einer Menge von 1 bis 4 Gew.-% enthalten. Möglich ist auch eine Stabilisierung mit einer Kombination cycloaliphatischer Glycidylester mit tertiären Estern der phosphorigen Säure.

Die Stabilisierung des chlorierten Paraffins erfolgt zweckmäßigerweise unmittelbar nach beendeter Chlorierung durch Einarbeitung des Stabilisators oder der Stabilisatoren in das noch warme Chlorierungsprodukt. Man erreicht auf diese Weise optimale Stabilitätsgrade.

Derartig stabilisierte chlorierte Paraffine ergeben als Lösemittel für die Farbbildner keine Schleierbildung der damit hergestellten Durchschlagpapiere. Die Fettaminoxethylate bewirken ferner auf Grund ihrer Grenzflächenaktivität eine gute Dispergierfähigkeit der Farbbildnerlösung in Wasser. Infolge der guten Löslichkeit der Fettaminoxethylate in chlorierten Paraffinen sind zusätzliche Solubilisierungsmittel nicht erforderlich. Ebenso kann durch die Grenzflächenaktivität der Fettaminoxethylate der Zusatz von Tensiden entfallen. Fettaminoxethylate vereinigen daher eine Reihe von Eigenschaften in einer Substanz und bringen daher einen erheblichen Fortschritt.

Die erfindungsgemäß stabilisierten chlorierten Paraffine können, wie in der Praxis meist üblich, mit einem anderen Lösemittel, beispielsweise mit einem Kohlenwasserstoff-Verdünnungsmittel, zum Beispiel einem Kerosintyp, vermischt werden.

Die Erfindung wird in den nachfolgenden Beispielen näher erläutert.

## Beispiele

In je 100 g mit Fettaninoxethylaten stabilisiertem chlorierten Paraffin wurden 0,2 g Farbbildner -Kristallviolettlacton - gelöst. Diese Lösung wurde über einen Zeitraum von 80 min auf 120 °C erhitzt. In Zeitabständen von 10, 15, 20, 30, 40, 50, 60 und 80 min wurden Proben entnommen, schnell auf Raumtemperatur gekühlt und die optische Dichte - Lichtabsorption - der Probe bei 605 nm in einer 1 cm-Küvette mit einem Spektralphotometer gegen eine Blindprobe des gleichen chlorierten Paraffin-Typs mit Fettaminoxethylat, aber ohne Farbbildner, die unter gleichen Bedingungen erhitzt worden war, gemessen. Am Ende der Versuchszeit von 80 min wurde außerdem die Jodfarbzahl der Farbbildnerlösung zur

Feststellung der Alterung des chlorierten Paraffins gemessen.

Eingesetzt wurden folgende chlorierte Paraffine:

A: chloriertes, im wesentlichen geradkettiges Paraffin mit 14-17 Kohlenstoffatomen und einem Chlorgehalt von ca. 52 Gew.-%, Handelsprodukt.

B: chloriertes, im wesentlichen geradkettiges Paraffin mit 14-17 Kohlenstoffatomen und einem Chlorgehalt von ca. Gew.-%, Handelsprodukt.

C: chloriertes, im wesentlichen geradkettiges Paraffin mit 14-17 Kohlenstoffatomen und einem Chlorgehalt von ca. 50 Gew.-%.

D: chloriertes, im wesentlichen geradkettiges Paraffin mit 10-13 Kohlenstoffatomen und einem Chlorgehalt von ca. 40 Gew.-%.

E: chloriertes, im wesentlichen geradkettiges Paraffin mit 10-13 Kohlenstoffatomen und einem Chlorgehalt von ca. 40 Gew.-%.

F: chloriertes, im wesentlichen geradkettiges Paraffin mit 14-17 Kohlenstoffatomen und einem Chlorgehalt von ca. 50 Gew.-%.

Die chlorierten Paraffine waren mit einem epoxidierten Sojabohnenöl oder einem cycloaliphatischen Diepoxid stabilisiert. Die verwendeten Fettaminoxethylate waren vom Typ Cocosfettaminoxethylat, Stearylaminoxethylat, Oleylaminoxethylat und Talgfettaminoxethylat mit 8 bis 25 Molekülen Ethylenoxid je Molekül Fettamin. Die erhaltenen Ergebnisse sind in den Tabellen 1-6 zusammengestellt. Die in den Tabellen bei den Fettaminoxethylaten jeweils angegebenen Ziffern geben die Anzahl der Moleküle Ethylenoxid je Molekül Fettamin an.

Tabelle 1

| chlorierter Paraffintyp: A<br>1,5 % epoxidiertes Sojaöl<br>Fettaminoxethylat: 1,5 % | | | | | | | | | | | |
|---|---|---|---|---|---|---|---|---|---|---|---|
| Fettaminoxethylat | | | | optische Dichte in % Absorption | | | | | | | Jodfarbzahl |
| Cocos- | Stearyl- | Oleyl- | Talg- | 10′ | 15′ | 20′ | 30′ | 40′ | 60′ | 80′ | |
| - | - | - | - | 6 | 20 | 34 | 46 | 65 | 75 | 85 | 20 |
| 5 | | | | 0 | 1 | 2 | 5 | 8 | 10 | 12 | 20 |
| 10 | | | | 0 | 0 | 1 | 3 | 5 | 9 | 12 | 20 |
| | | 8 | | 0 | 1 | 1 | 2 | 3 | 6 | 8 | 20 |
| | | 12 | | 0 | 0 | 1 | 2 | 4 | 6 | 8 | 20 |
| | | 20 | | 1 | 1 | 1 | 2 | 4 | 6 | 7 | 15 |
| | | | 10 | 0 | 0 | 0 | 0 | 2 | 4 | 6 | 10 |
| | | | 15 | 0 | 0 | 0 | 1 | 3 | 5 | 7 | 15 |
| | 8 | | | 0 | 0 | 0 | 0 | 1 | 5 | 8 | 20 |
| | 15 | | | 0 | 0 | 1 | 1 | 3 | 5 | 6 | 10 |

Tabelle 2

| chlorierter Paraffintyp: B<br>1,5 % epoxidiertes Sojaöl<br>Fettaminoxethylat: 1,5 % | | | | | | | | | | | |
|---|---|---|---|---|---|---|---|---|---|---|---|
| Fettaminoxethylat | | | | optische Dichte in % Absorption | | | | | | | Jodfarbzahl |
| Cocos- | Stearyl- | Oleyl- | Talg- | 10′ | 15′ | 20′ | 30′ | 40′ | 60′ | 80′ | |
| - | - | - | - | 7 | 23 | 37 | 48 | 62 | 70 | 75 | 15 |
| 5 | | | | 0 | 0 | 0 | 1 | 2 | 5 | 7 | 10 |
| 10 | | | | 0 | 0 | 0 | 1 | 2 | 3 | 6 | 15 |
| | | 8 | | 0 | 0 | 0 | 1 | 1 | 2 | 4 | 10 |
| | | 12 | | 0 | 0 | 0 | 1 | 2 | 2 | 3 | 10 |
| | | 20 | | 0 | 0 | 0 | 1 | 2 | 2 | 3 | 10 |
| | | | 10 | 0 | 0 | 0 | 1 | 2 | 3 | 5 | 15 |
| | | | 15 | 0 | 0 | 1 | 2 | 3 | 4 | 6 | 15 |
| | 8 | | | 0 | 0 | 0 | 1 | 1 | 3 | 4 | 15 |
| | 15 | | | 0 | 0 | 1 | 2 | 3 | 4 | 5 | 15 |

Tabelle 3

| chlorierter Paraffintyp: C<br>1 % epoxidiertes Harz<br>Fettaminoxethylat: 1,5 % | | | | | | | | | | | |
|---|---|---|---|---|---|---|---|---|---|---|---|
| Fettaminoxethylat | | | | optische Dichte in % Absorption | | | | | | | Jodfarbzahl |
| Cocos- | Stearyl- | Oleyl- | Talg- | 10′ | 15′ | 20′ | 30′ | 40′ | 60′ | 80′ | |
| - | - | - | - | 6 | 20 | 33 | 45 | 60 | 68 | 70 | 20 |
| 5 | | | | 0 | 1 | 1 | 2 | 3 | 6 | 8 | 10 |
| 10 | | | | 0 | 0 | 1 | 3 | 4 | 7 | 9 | 20 |
| | | 8 | | 0 | 1 | 1 | 3 | 4 | 6 | 9 | 20 |
| | | 12 | | 0 | 0 | 1 | 1 | 2 | 4 | 6 | 20 |
| | | 20 | | 0 | 0 | 0 | 1 | 1 | 3 | 4 | 12 |
| | | | 10 | 0 | 0 | 0 | 0 | 1 | 4 | 6 | 10 |
| | | | 15 | 0 | 0 | 0 | 0 | 0 | 3 | 5 | 10 |
| | 8 | | | 0 | 0 | 0 | 1 | 1 | 4 | 6 | 10 |
| | 15 | | | 1 | 1 | 1 | 1 | 1 | 4 | 6 | 10 |

Tabelle 4

| chlorierter Paraffintyp: D<br>1,5 % epoxidiertes Sojaöl<br>Fettaminoxethylat: 1,5 % | | | | | | | | | | | |
|---|---|---|---|---|---|---|---|---|---|---|---|
| Fettaminoxethylat | | | | optische Dichte in % Absorption | | | | | | | Jodfarbzahl |
| Cocos- | Stearyl- | Oleyl- | Talg- | 10′ | 15′ | 20′ | 30′ | 40′ | 60′ | 80′ | |
| - | - | - | - | 10 | 30 | 42 | 60 | 73 | 80 | 85 | 10 |
| | | 20 | | 0 | 0 | 0 | 0 | 1 | 2 | 3 | 5 |

Tabelle 5

| chlorierter Paraffintyp: E<br>1 % epoxidiertes Harz<br>Fettaminoxethylat: 1,5 % | | | | | | | | | | | |
|---|---|---|---|---|---|---|---|---|---|---|---|
| Fettaminoxethylat | | | | optische Dichte in % Absorption | | | | | | | Jodfarbzahl |
| Cocos- | Stearyl- | Oleyl- | Talg- | 10′ | 15′ | 20′ | 30′ | 40′ | 60′ | 80′ | |
| - | - | - | - | 9 | 27 | 40 | 58 | 71 | 83 | 85 | 10 |
| | | 20 | | 0 | 0 | 0 | 0 | 0 | 1 | 1 | 3 |

Tabelle 6

| chlorierter Paraffintyp: F<br>1,5 % epoxidiertes Harz<br>Fettaminoxethylat: 1,5 % | | | | | | | | | | | |
|---|---|---|---|---|---|---|---|---|---|---|---|
| Fettaminoxethylat | | | | optische Dichte in % Absorption | | | | | | | Jodfarbzahl |
| Cocos- | Stearyl- | Oleyl- | Talg- | 10′ | 15′ | 20′ | 30′ | 40′ | 60′ | 80′ | |
| - | - | - | - | 9 | 27 | 42 | 57 | 73 | 86 | 88 | 20 |
| 5 | | | | 0 | 0 | 1 | 2 | 4 | 8 | 11 | 20 |
| 10 | | | | 0 | 0 | 1 | 1 | 2 | 5 | 8 | 12 |
| | | 8 | | 0 | 0 | 0 | 0 | 0 | 4 | 7 | 12 |
| | | 12 | | 0 | 0 | 0 | 0 | 1 | 4 | 7 | 12 |
| | | 20 | | 0 | 0 | 0 | 1 | 1 | 3 | 5 | 10 |
| | | | 10 | 0 | 0 | 0 | 1 | 1 | 4 | 7 | 12 |
| | | | 15 | 0 | 0 | 0 | 0 | 1 | 4 | 7 | 12 |
| | 8 | | | 0 | 0 | 0 | 1 | 1 | 3 | 6 | 10 |
| | 15 | | | 0 | 0 | 0 | 0 | 1 | 4 | 7 | 12 |

## Ansprüche

1. Verfahren zum Stabilisieren eines chlorierten, im wesentlichen geradkettigen Paraffins mit 7 bis 35 Kohlenstoffatomen und einem Chlorgehalt von 10 bis 72 Gew.-%, dadurch gekennzeichnet, daß man dem chlorierten Paraffin 0,1 bis 3 Gew.-%, bezogen auf das chlorierte Paraffin, mindestens eines Fettaminoxethylats zusetzt.
2. Verfahren nach Anspruch 1, dadurch gekennzeichnet, daß das Fettaminoxethylat auf Cocosfettamin, Stearylamin, Oleylamin oder Talgfettamin basiert.
3. Verfahren nach Anspruch 1, dadurch gekennzeichnet, daß das Fettaminoxethylat ein Reaktionsprodukt von je 1 mol Fettamin mit 2 bis 30 mol Ethylenoxid ist.
4. Verfahren nach Anspruch 1, dadurch gekennzeichnet, daß dem chlorierten Paraffin zusätzlich 1 bis 4 Gew.-%, bezogen auf das chlorierte Paraffin, eines Epoxidstabilisators zugesetzt wird.
5. Chloriertes, im wesentlichen geradkettiges Paraffin mit 7 bis 35 Kohlenstoffatomen und einem Chlorgehalt von 10 bis 72 Gew.-%, enthaltend 0,1 bis 3 Gew.-%, bezogen auf das chlorierte Paraffin, mindestens eines Fettaminoxethylats.
6. Verwendung des chlorierten Paraffins nach Anspruch 5 zur Herstellung chemisch reagierender Durchschreibepapiere.

Europäisches Patentamt

# EUROPÄISCHER RECHERCHENBERICHT

Nummer der Anmeldung

EP 90116038.2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl.⁵) |
|---|---|---|---|
| A | EP - A1 - 0 222 489 (ICI) * Zusammenfassung; Ansprüche 1,3-5; Tabelle 1 * -- | 1,4-6 | C 07 C 17/42 B 41 M 5/165 |
| A | DE - B1 - 2 801 956 (HOECHST) * Ansprüche 1,2,5; Versuche Nr. 2b,3a * -- | 1,4,5 | |
| A | DE - C - 965 397 (FARBWERKE HOECHST) * Gesamt * ---- | 1,5 | |

RECHERCHIERTE SACHGEBIETE (Int Cl.⁵)

C 07 C 17/00
B 41 M 5/00
C 07 C 19/00

Der vorliegende Recherchenbericht wurde fur alle Patentanspruche erstellt.

| Recherchenort | Abschlußdatum der Recherche | Prufer |
|---|---|---|
| WIEN | 26-11-1990 | KÖRBER |

KATEGORIE DER GENANNTEN DOKUMENTEN

X : von besonderer Bedeutung allein betrachtet
Y : von besonderer Bedeutung in Verbindung mit einer anderen Veroffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument